# EUROPEAN PATENT APPLICATION

(11) **EP 1 683 489 A1**
(43) Date of publication of application: **26.07.2006**
(21) Application number: 06001398.4
(22) Date of filing: 24.01.2006
(51) Int. Cl.: A61B 17/02

(54) **Illuminated surgical retractor**

(30) Priority: 25.01.2005 US 42496
(71) Applicant: Zimmer Technology, Inc., Chicago, Illinois 60606 (US)
(72) Inventor: Acker, Dean M. J., Warsaw, IN 46582 (US); Krebs, Robert D., Warsaw, IN 46580 (US)
(74) Representative: Manitz, Finsterwald & Partner GbR

(57) **Abstract**

The invention describes an apparatus, in particular for illuminated retraction of a surgical cavity, comprising a surgical retractor and a light conduit coupled to the retractor, the light conduit terminating at a light emitting end adjacent the retractor or within an opening of the retractor.

## Description

### FIELD OF THE INVENTION

The present invention relates generally to the field of medicine, and, more particularly, to illuminated surgical retractors.

### BACKGROUND

In general, surgical retractors are used to push, pull, hold and/or fold skin, flesh and/or other tissue away from a site where a surgical operation or other intervention is being performed. Retractors expand the cavity or working area around the site, providing more room in which to maneuver operating and diagnostic tools. Retractors have also been used to facilitate separation of various tissues from architectures proximal to the surgical site, thereby improving access to and visibility of the site.

Historically, surgical retractors have been comprised of two main parts: a body or handle portion and an insertion portion or insert. The body is typically held by an operator when manipulating the retractor or coupled to a support frame that may include weights or mechanisms designed to facilitate desired movements and hold the retractor in place. The insert is suitably configured to move or grasp the desired tissues. For example, by putting a hook-shaped insert into a surgical cavity and then rotating it, surrounding tissues may be snared and then pulled away from the working environment. Not surprisingly, a single size and shape for retractors has not been practical. Indeed, a wide variety of geometries has been developed for different surgical procedures. Retractors have also been used in conjunction with external lighting systems wherein the retractor holds the body tissue out of the way while the lighting system concurrently illuminates the body cavity. However, relying on directed lighting external to a surgical cavity can be problematic due to difficulties in projecting the light in the required direction and shadows that may be cast onto the operating field. Moreover, separate retracting and lighting systems may be frustrating for an operator who is forced to manipulate both systems simultaneously, and various problems may arise as separate lighting and retracting tools get in the way of each other and cross paths with other equipment in the operating room.

Some retractor designs have sought to integrate retracting and lighting functions into a single device. However, the various complex ways of housing light sources and delivering light to the inserts in many of these illuminated or lit retractors have produced limited retractor geometries, bulky and/or heavy handles and inserts, and/or maintenance issues. Furthermore, some illuminated retractors have tended to emit narrow spot beams of light directed to rather small locations of the operating site. As such an illuminated retractor is moved, as is typically necessary to perform its very retracting function, the narrow spot beam of light is concurrently (and undesirably) moved around the cavity in various directions. While some other illuminated retractors have been designed to provide more diffuse lighting, historical diffusion techniques such as frost or ground lenses can produce light losses that reduce the overall intensity or brightness (relative to the light source) of any light that is eventually delivered to the cavity. Moreover, depending on the application, sometimes the availability of a directed beam may be desirable.

Consequently, the competing needs for variety in size and geometry, directed lighting and diffuse lighting, and simplicity have tended to limit the effectiveness of historical illuminated retractors.

### SUMMARY

The present invention provides an apparatus including a surgical retractor and a light conduit.

In one aspect of the invention, an apparatus for illuminated retraction of a surgical cavity includes a retractor having a portion insertable into a surgical cavity and a light conduit. The portion has a first surface for abutting the margins of the surgical cavity and a second surface opposite the first surface facing away from the margins abutted by the first surface. The second surface includes an opening. The light conduit includes a light emitting end having a longitudinal axis. The light emitting end is disposed within the opening in the second surface and has a shape corresponding to the shape of the second surface.

In another aspect of the invention, an apparatus is provided having a surgical retractor and a light conduit. The retractor includes a retractor surface. The light conduit is releasably coupled to the retractor. The conduit includes a sheath having an outer wall terminating at an angled end portion and an opening extending through the sheath wall at the angled end. A plurality of light transmitting filaments is disposed within the sheath. The filaments terminate in an end portion having a longitudinal axis and an end face normal to the axis and contained within the sheath. The end portion is fully recessed within the retractor surface.

In another aspect of the invention, an apparatus is provide including a surgical retractor and a light conduit coupled to the retractor. The light conduit includes a plurality of light transmitting filaments. The filaments terminate in a stair-step arrangement at a light emitting end adjacent the retractor.

In another aspect of the invention, an apparatus includes a surgical retractor and a light conduit coupled to the retractor. The light conduit includes at least one light transmitting filament terminating at a light emitting end having a longitudinal axis. The at least one filament defines a planar surface at the light emitting end oblique to the longitudinal axis and positioned within the opening.

### BRIEF DESCRIPTION OF THE DRAWINGS

Various examples of the present invention will be discussed with reference to the appended drawings. These drawings depict only illustrative examples of the invention and are not to be considered limiting of its scope.

FIG. 1 shows a side (or profile) view of an exemplary apparatus according to the present invention;

FIG. 2 shows a top view of the exemplary apparatus;

FIG. 3 shows a laterally exploded top view of the exemplary apparatus;

FIG. 4 shows a bottom view of the exemplary apparatus;

FIG. 5 shows a laterally exploded bottom view of the exemplary apparatus;

FIG. 6 shows a side sectional view of the apparatus of FIG. 1, taken along line 6-6 of FIG.2, showing an alternate tip configuration;

FIG. 7 shows a detail side sectional view of the apparatus of FIG. 1 showing an alternate tip configuration;

FIG. 8 shows a detail top view of the apparatus of FIG. 7;

FIG. 9 shows a detail side sectional view of the apparatus of FIG. 1 showing an alternate tip configuration;

FIG. 10 shows a detail top view of the apparatus of FIG. 9;

FIG. 11 shows a detail side sectional view of the apparatus of FIG. 1 showing an alternate tip configuration;

FIG. 12 shows a detail top view of the apparatus of FIG. 11.

### DESCRIPTION OF THE ILLUSTRATIVE EXAMPLES

It is noted that as used throughout this disclosure and the claims, the terms "finger-releasable," "finger-releasably," and the like mean separable by a human hand(s), finger(s), and/or thumb(s)―without tools; whereas, the terms "releasable," "releasably," and the like mean separable with or without tools.

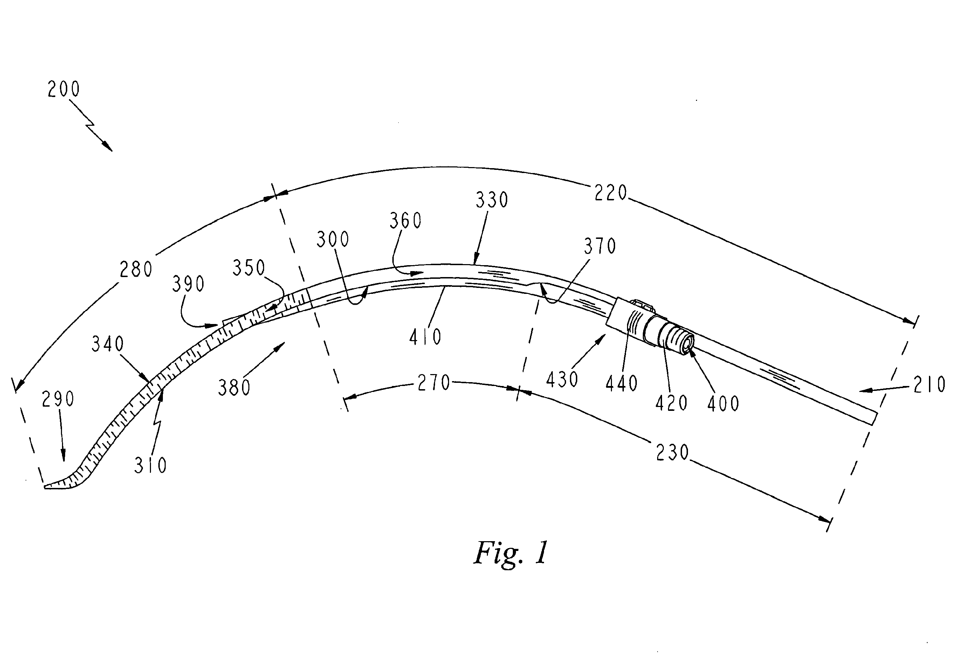

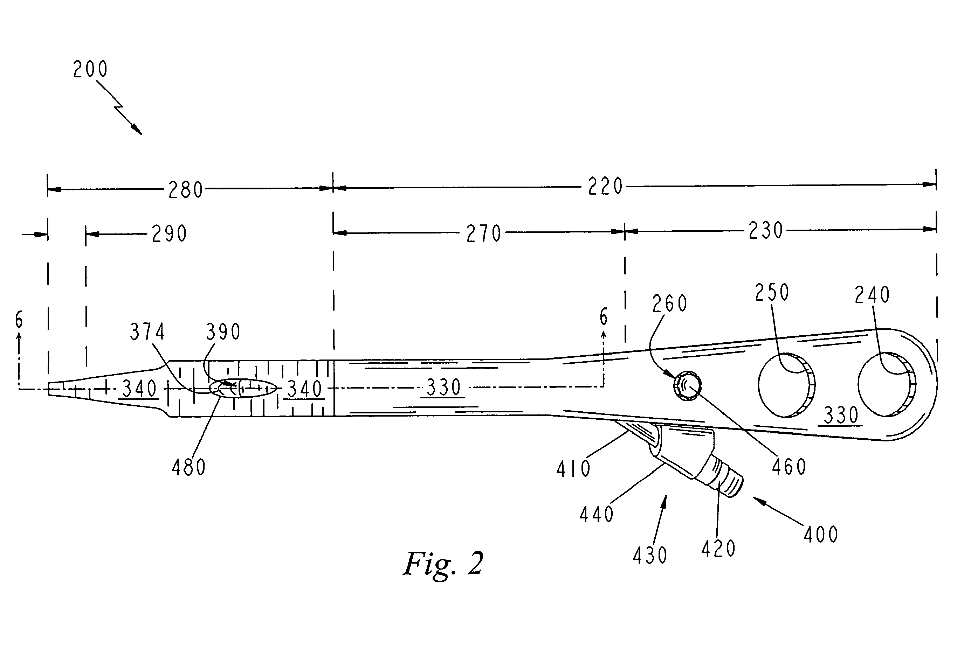

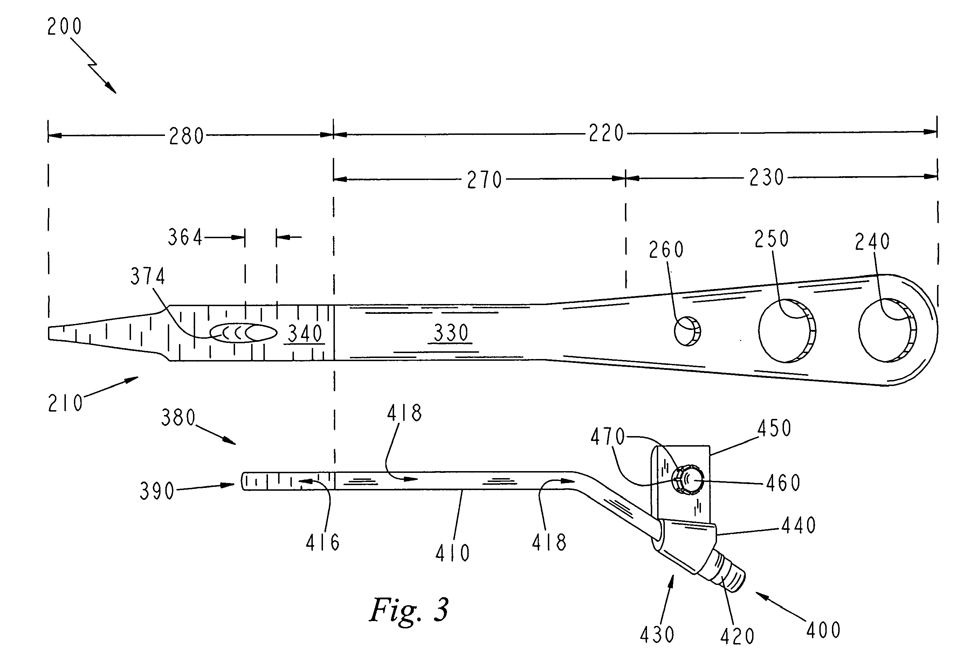

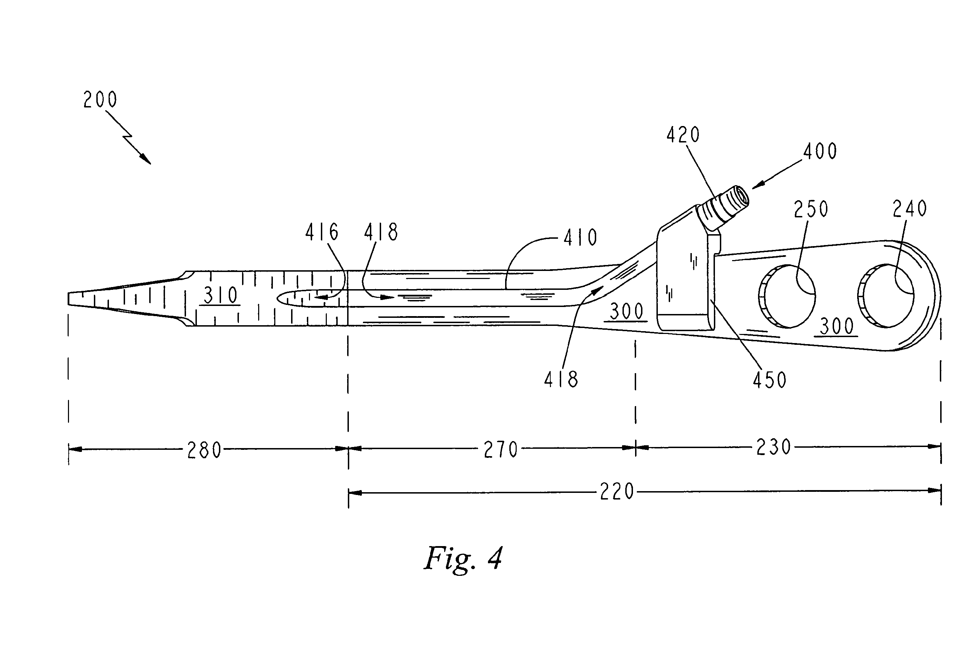

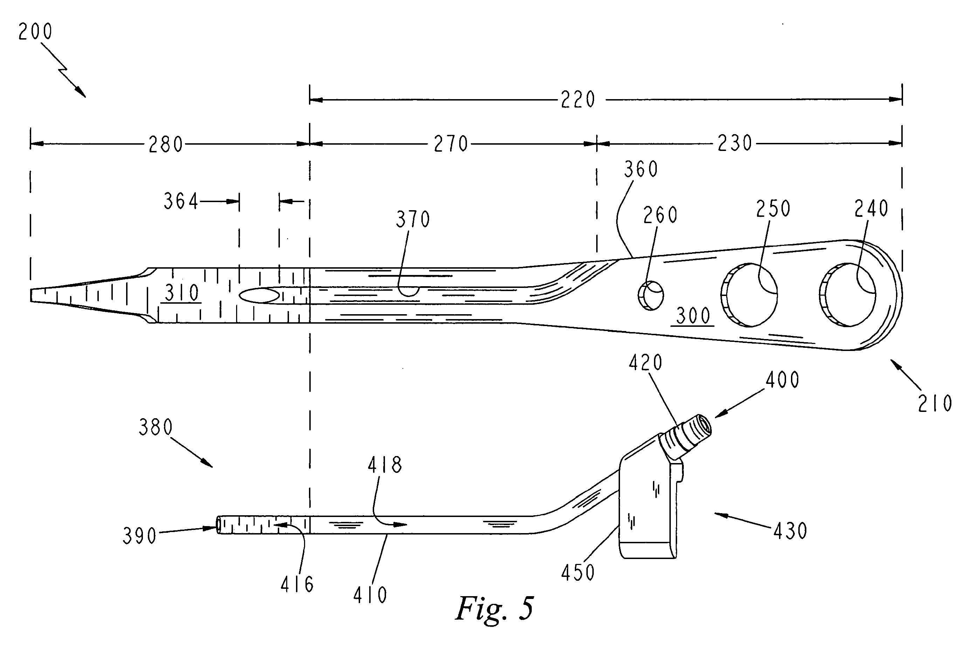

FIG. 1 shows a side (or profile) view of an exemplary apparatus 200 according to the present invention. Apparatus 200 is of suitable size and weight for manipulation by hand and includes a surgical retractor 210 that is suitable for sterilization in an autoclave. In the exemplary embodiment, surgical retractor 210 is made from stainless steel. In alternative embodiments, surgical retractor 210 may be made from high temperature plastic or any other material or combination of materials suitable for use in surgical procedures. Surgical retractor 210 includes a body portion 220. Portion 220 includes a generally planar portion 230 defining an aperture or hole 240, an aperture or hole 250, and an aperture or hole 260 (see FIG. 2, FIG. 3, and FIG. 5), and further includes a generally arcuate or curved portion 270 extending from portion 230. Surgical retractor 210 further includes a generally arcuate or curved insertion portion 280 extending from portion 270. Portion 280 includes a generally arcuate or curved end portion 290. Portion 220 includes a lackluster bottom surface 300 and portion 280 includes a lustrous bottom surface 310 (see also FIG. 4. and FIG. 5). Also, portion 220 includes a lackluster top surface 330, while portion 280 includes a lustrous top surface 340. Portion 280 also includes a lustrous side surface 350 extending between surface 310 and surface 340, and further includes an opposing lustrous side surface (not shown). Meanwhile, portion 220 also includes a lackluster side surface 360 extending between surface 300 and surface 330, and further includes an opposing lackluster side surface (not shown). In the exemplary embodiment, the lustrous surfaces (e.g., 340, 350, 310) are produced by suitably color buffing portion 280 in a known manner and the lackluster surfaces (e.g., 330, 360, 300) are produced by suitably mass finishing portion 220 in a known manner. Additionally, portion 280 defines a generally ovular aperture or hole 364 extending between surface 310 and surface 340 (see FIG. 3 and FIG. 5). Surface 300, surface 310, and surface 360 define a groove or channel 370 extending between surface 360 and hole 364 (see FIG. 5), while surface 340 defines a groove or channel 374 that also communicates with hole 364 (see FIG. 2).

Apparatus 200 also includes a light conduit 380 having a side-view geometry or profile substantially conforming to that of surgical retractor 210. Conduit 380 includes an end 390, an end 400, and an optional casing or sheath 410. Sheath 410 extends through channel 370 and hole 364 such that end 390 protrudes from an intermediate portion of channel 374 (see also FIG. 2, FIG. 4, and FIG. 5). Further, sheath 410 includes a lustrous outer surface 416 extending from end 390 and a lackluster outer surface 418 extending from surface 416 to end 400 (see FIG. 3, FIG. 4, and FIG. 5).

In general, conduit 380 is suitable for use in surgical procedures and configured to transmit externally generated light from end 400 to end 390. Accordingly, conduit 380 includes one or more fiber optic cables and/or any other suitable light transmitting materials. The light transmitting materials may be in the form of a single cable or filament such as a glass or plastic rod. The filament may be molded or otherwise formed into a desired cross-sectional and longitudinal shape. The single filament may alone form the conduit 380 or the single filament may be housed in optional sheath 410 to protect the filament, provide additional rigidity or flexibility, and/or improve the light transmission and/or dispersion of the conduit 380. Alternatively, the light transmitting materials may be in the form of a plurality of filaments such as a plurality of glass or plastic filaments. The plurality of filaments may be loosely gathered and constrained in a desired shape by placing them within optional sheath 410. The filaments may also be formed into a self-supporting structure such as by adhering them to one another with an adhesive and/or by fusing them with heat and/or pressure. The multifilament structure may then be optionally housed in sheath 410. The conduit 380 includes a coupling member 420 fixedly housing a portion of sheath 410 proximal to end 400. Member 420 is configured in a known manner for finger-releasably coupling conduit 380 to an external light source.

In the exemplary embodiment, conduit 380 is reusable and suitable for sterilization in an autoclave. Accordingly, sheath 410 is a rigid stainless steel pipe with lustrous outer surface 416 produced by suitable color buffing and lackluster outer surface 418 produced by suitable mass finishing. In alternative embodiments, the various components of conduit 380 may be made from high temperature plastic and/or any other material or combination of materials suitable for use in surgical procedures and sterilization in an autoclave, and sheath 410 may or may not be flexible. It is noted, however, that in some alternative embodiments conduit 380 may be disposable and, accordingly, in such alternative embodiments all of the components of conduit 380 may be made of relatively inexpensive low temperature acrylics or polymers.

Apparatus 200 further includes a bracket 430 that finger-releasably couples conduit 380 to surgical retractor 210. Bracket 430 includes a sleeve portion 440 fixedly housing a portion of sheath 410 of conduit 380 proximal to end 400 and distal to end 390 (see also FIG. 2 and FIG. 3). Bracket 430 further includes a generally planar flange portion 450 extending laterally underneath portion 230 of surgical retractor 210 proximal to surface 300 (see FIG. 3, FIG. 4, and FIG. 5). Bracket 430 also includes a button or peg 460, having a plurality of slits 470 therein, extending upward from flange portion 450 and snugly fitted into hole 260 of surgical retractor 210 such that peg 460 (and thus bracket 430 and conduit 380) is finger-releasably coupled to surgical retractor 210 (see FIG. 2 and FIG. 3). It should be appreciated, however, that various components in alternative embodiments may include a screw/socket arrangement, a lever operated latch, or any other suitable alternative coupling or couplings for releasably coupling conduit 380 to surgical retractor 210, including (in some embodiments) a coupling that releasably couples conduit 380 to surgical retractor 210 but does not finger-releasably couple conduit 380 to surgical retractor 210.

FIG. 2 shows a top view of exemplary apparatus 200. Hole 240, hole 250, hole 260, and channel 374 are discernible in FIG. 2. Additionally, FIG. 2 shows that channel 374 includes a generally ovular rim 480. Portion 220, portion 230, portion 270, portion 280, portion 290, surface 330, surface 340, end 390, end 400, sheath 410, member 420, bracket 430, portion 440, and peg 460 are discussed above in connection with FIG. 1.

FIG. 3 shows a laterally exploded top view of exemplary apparatus 200. Among other things, surgical retractor 210 (including portion 220, portion 230, hole 240, hole 250, hole 260, portion 270, portion 280, surface 330, surface 340, hole 364, and channel 374), conduit 380 (including end 390, end 400, sheath 410, surface 416, surface 418, and member 420), and bracket 430 (including portion 440, portion 450, peg 460, and slits 470)―all discussed above in connection with FIG. 1―are discernable in FIG. 3.

FIG. 4 shows a bottom view of exemplary apparatus 200. Among other things, portion 220, portion 230, portion 270, portion 280, hole 240, hole, 250, surface 300, surface 310, end 400, sheath 410, surface 416, surface 418, and member 420―all discussed above in connection with FIG. 1―are discernable in FIG. 4.

FIG. 5 shows a laterally exploded bottom view of exemplary apparatus 200. Among other things, surgical retractor 210 (including portion 220, portion 230, hole 240, hole 250, hole 260, portion 270, portion 280, surface 300, surface 310, surface 360, hole 364, and channel 370), conduit 380 (including end 390, end 400, sheath 410, surface 416, surface 418, and member 420), and bracket 430 (including portion 450)―all discussed above in connection with FIG. 1―are discernable in FIG. 5.

In operation of exemplary apparatus 200, member 420 of light conduit 380 is coupled to a suitable external light source and insertion portion 280 of surgical retractor 210 is inserted into a surgical cavity. Body portion 220 is used to grasp and manipulate surgical retractor 210 as desired. It should be appreciated that the low profile and light weight of exemplary apparatus 200 facilitates its manipulation. In any event, light from the external source is emitted from end 390 of light conduit 380. Channel 374 focuses some of this light into somewhat of a spotlight like beam. Insertion portion 280 is suitably maneuvered to direct the focused light into a desired portion of the surgical cavity. Meanwhile, one or more of the lustrous surfaces (e.g., 340, 350, 310) also reflect a portion of the light present in the surgical cavity, thereby dispersing some of the light to generally illuminate another portion or portions of the surgical cavity at a somewhat lower intensity than the area illuminated by the focused light. Consequently, general or somewhat diffuse lighting of the surgical cavity is provided concurrently with more focused lighting of relatively higher intensity.

For additional dispersion and/or additional focused lighting, additional surgical retractor 210 and/or apparatus 200 are inserted into the surgical cavity as desired. In such cases, various lustrous surfaces of the various surgical retractor 210 may cooperate somewhat to reflect light amongst themselves, thereby enhancing the dispersive effect while maintaining the availability of one or more directable beams.

To facilitate cleaning of apparatus 200 or use of surgical retractor 210 without light conduit 380, conduit 380 is released from surgical retractor 210 by pushing peg 460 of bracket 430 out of hole 260 of surgical retractor 210 with a finger or thumb, and conduit 380 is removed from channel 370 and channel 374 by moving bracket 430 generally down and away from surgical retractor 210 and by pulling bracket 430 generally away from hole 364 such that end 390 of conduit 380 is withdrawn from channel 374 through hole 364, thereby separating conduit 380 and bracket 430 from surgical retractor 210.

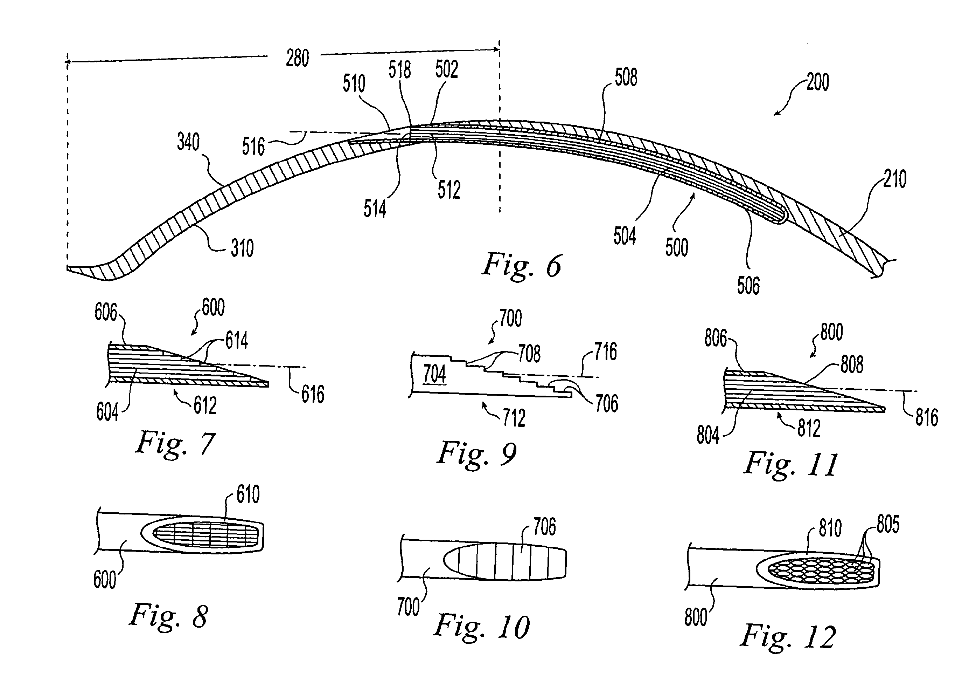

FIGS. 6-12 show alternative configurations for the tip of the light conduit in which the light conduit terminates at an oblique end so that the end lies flush with, or recessed below, the top surface 340 of the insertion portion 280 of the retractor 210. The oblique end may be generally planar or it may be a simple or complex curve. Preferably, the end corresponds to the shape of the top surface 340 of the retractor 210 adjacent the hole 364 where the end resides. If the top surface 340 is curved but of a relatively large radius, an angled planar end will be a close approximation of this surface and may be simpler and less expensive to manufacture. By having the conduit end flush, or below flush, with the top surface 340 of the retractor 210, the apparatus 200 presents a smooth surface such that other surgical components, including instruments and implants, will not catch on the apparatus 200 as they are passed into and out of the surgical site. Likewise, this arrangement provides the largest possible opening between the retractor 210 and the rest of the surgical site. In each of these examples, a sheath may be included if it provides more desirable light transmission properties, more desirable flexibility or rigidity, and/or protection of the conduit. Also, the sheath may be omitted to lower cost and/or to maximize the amount of light transmission area within a given available space. The conduits in these examples may include one or more light transmitting filaments for transmitting light to the end.

In typical use, the insertion portion 280 of the retractor 210 is inserted into a surgical cavity with the bottom surface 310 abutting the margins of the surgical cavity to retract the margins and enlarge the cavity opening. The top surface 340 faces away from the margins of the cavity. The light conduit emits light through the hole 364 in the top surface 340 to illuminate the surgical cavity.

The exemplary conduit 500 of FIG. 6 includes a plurality of light transmitting filaments 504 contained within an optional sheath 506. The sheath 506 includes a wall 508 terminating at an angled light emitting end 502. An angled opening 510 extends through the sheath wall 508 to allow light to exit the sheath 506 at the end 502. The filaments 504 terminate in a cylindrical end portion 512 having a longitudinal axis 516 and an end face 514 normal to the axis 516 and contained within the sheath 506. However, it is anticipated that the end portion 512 and/or the conduit 500 may have non-cylindrical shapes as well such as rectangular, oval, non-rectangular polygonal, and/or other suitable shapes. The end portion 512 terminates at the distal edge 518 of opening 510 so that the filaments 504 do not extend beyond the outer wall 508 of the sheath 506 and consequently so that they do not extend beyond the top surface 340 of the retractor 210. Light is projected from the end portion 512 to illuminate the surgical site. By recessing the light transmitting filaments 504 within the sheath 506, the sheath 506 can include an angled end while maintaining a normal end face 514 on the filaments 504 to preserve maximum light transmission out of the conduit 500 along the axis 516.

The exemplary conduit 600 of FIGS. 7 and 8 includes a plurality of light transmitting filaments 604 contained within an optional sheath 606. The sheath terminates in an angled opening 610 similar to the configuration of FIG. 6. The filaments 604 terminate in an elongate end portion 612 having a longitudinal axis 616. The filaments 604 are terminated in a cascading or stair-step configuration so that each filament 604 extends as far as possible without extending beyond the opening 610. Each filament 604 terminates in an end face 614 normal to the axis 616 for maximum light transmission out of the conduit 600 along the axis 616. The filament ends 614 may more or less closely approximate the shape of the top surface 340 of the retractor 210 depending on how fine the individual filaments 604 are. An area within the surgical site that is illuminated beyond the end 602 of the conduit 600 will experience both relatively more diffuse and relatively more focused light from the conduit 600 of FIGS. 7 and 8. The light exiting from filaments 604 terminating closer to the end 602 disperses less by the time it reaches the surgical site than light exiting from filaments 604 terminating further from the end 602. Therefore, general or relatively more diffuse lighting of the surgical site is provided by the filaments 604 terminating further from the end 602 and spot or relatively more focused lighting of the surgical site is provided by the filaments 604 terminating closer to the end 602.

The exemplary conduit 700 of FIGS. 9 and 10 includes a single light transmitting filament 704 for transmitting light to an elongate end portion 712 having a longitudinal axis 716. The filament 704 is terminated by forming a series of stair steps 706 each having an end face 708 normal to the longitudinal axis 716. The end 702 may more or less closely approximate the shape of the top surface 340 of the retractor 210 depending on how finely or coarsely the stair steps 706 are formed. The steps may be equal to approximate a planar angled surface or they may be unequal to approximate a curved surface. The normal end faces 708 provide for maximum light transmission out of the conduit along the axis 716 while the stair step arrangement provides an illumination pattern similar to that of the example of FIGS. 7 and 8. The exemplary embodiment of FIGS. 9 and 10 omits the optional sheath shown in the prior examples.

The exemplary conduit 800 of FIGS. 11 and 12 includes a plurality of light transmitting filaments 804 contained within an optional sheath 806. The sheath 806 terminates in an angled opening 810 similar to the configuration of FIG. 6. The filaments 804 terminate in an elongated end portion 812 having a longitudinal axis 816. However, in the exemplary embodiment of FIGS. 11 and 12, the filaments 804 each terminate at an angled surface 805 corresponding to the angle of the opening 810. For example, the filaments 804 may be cut and ground at an angle. The resulting conduit 800 will transmit less light along the axis 816 than the prior examples because the angled surfaces 805 tend to reflect more light back into the conduit 800 than do the normal surfaces of the preceding examples. However, the conduit 800 of FIGS. 11 and 12 can provide a continuous, smooth surface 808 flush with the top surface 340 of the retractor 210 so that there are no edges, holes, or projections to interfere with the surgical procedure. A single filament 804 could be provided having a smoothly angled end surface 805 as well.

The foregoing description of the invention is illustrative only, and is not intended to limit the scope of the invention to the precise terms set forth. Further, although the invention has been described in detail with reference to certain illustrative embodiments, variations and modifications exist within the scope and spirit of the invention as described and defined in the following claims.

## Claims

1. Apparatus, in particular for illuminated retraction of a surgical cavity, comprising:
a surgical retractor (210); and
a light conduit (380, 500, 600, 700, 800) coupled to the retractor (210), the light conduit terminating at a light emitting end (390, 502) adjacent the retractor (210) or within an opening (364) of the retractor (210).

2. Apparatus according to claim 1,
**characterized in that**
the light conduit comprises a plurality of light transmitting filaments (504, 604, 804).

3. Apparatus according to claim 2,
**characterized in that**
the filaments (604) terminate in a stair-step arrangement (614) at the light emitting end.

4. Apparatus according to claim 2 or 3,
**characterized in that**
the retractor comprises a retractor surface and the filaments (604) terminate in a stair-step arrangement (614) corresponding approximately to the shape of the retractor surface (340).

5. Apparatus according to any of claims 2 to 4,
**characterized in that**
the light emitting end (502) has a longitudinal axis (516), the filaments (504) each terminating in an end face (514) normal to the longitudinal axis (516), the light conduit (500) further comprising a sheath (506) surrounding the filaments (504), the sheath (506) terminating at the light emitting end (502) in an angled opening (510) oblique to the longitudinal axis (516).

6. Apparatus according to claim 1,
**characterized in that**
the surgical retractor (210) has a portion (280) insertable into the surgical cavity, the portion (280) having a first surface (310) for abutting the margins of the surgical cavity and a second surface (340) opposite the first surface (310) facing away from the margins abutted by the first surface (310), the second surface (340) defining an opening (364); and
the light emitting end (390) of the light conduit (380, 600, 700, 800) has a longitudinal axis (616, 716, 816), the light emitting end (390) being disposed within the opening (364) generally flush with the second surface (340) and having a shape corresponding to the shape of the second surface (340), the light emitting end (390) emitting light through the opening (364) along the longitudinal axis (616, 716, 816).

7. Apparatus according to claim 6,
**characterized in that**
the light conduit (380, 700) includes a light transmitting material comprising a single filament (704).

8. Apparatus according to claim 7,
**characterized in that**
the light conduit (380) includes a sheath (410) surrounding the filament.

9. Apparatus according to claim 6,
**characterized in that**
the light conduit (380) includes a light transmitting material comprising a plurality of filaments (604, 804).

10. Apparatus according to claim 9,
**characterized in that**
the filaments (604, 804) are formed into a self-supporting structure by adhering the filaments to one another.

11. Apparatus according to claim 9 or 10,
**characterized in that**
the light conduit includes a sheath (410, 606, 806) surrounding the filaments (604, 804).

12. Apparatus according to any of claims 9 to 11,
**characterized in that**
the light conduit includes a rigid pipe (410) surrounding the filaments, the surgical retractor (210) includes a channel (370) communicating with the opening (364) in the second surface (340), and at least a portion of the rigid pipe is recessed in the channel (370).

13. Apparatus according to any of claims 6 to 12,
**characterized in that**
the light conduit (380, 600, 700, 800) is releasably coupled to the retractor (210).

14. Apparatus according to any of claims 6 to 13,
**characterized in that**
the light emitting end (390) has a generally curved shape.

15. Apparatus according to any of claims 6 to 13,
**characterized in that**
the light emitting end (390) has a generally planar shape oblique to the longitudinal axis (816).

16. Apparatus according to any of claims 6 to 15,
**characterized in that**
the light conduit (380, 600, 700, 800) passes through the retractor (210) from the first surface (310) to the second surface (340).

17. Apparatus according to any of claims 6 to 16,
**characterized in that**
the conduit (600) comprises a plurality of light transmitting filaments (604), the filaments (604) terminating at the light emitting end in a stair-step arrangement (614) corresponding approximately to the shape of the second surface (340) of the retractor (210).

18. Apparatus according to any of claims 6 to 16,
**characterized in that**
the conduit (700) comprises a single light transmitting filament (704), the filament (704) terminating at the light emitting end in a plurality of steps (706) corresponding approximately to the shape of the second surface (340) of the retractor.

19. Apparatus according to any of claims 6 to 16,
**characterized in that**
the conduit (800) comprises a bundle containing a plurality of filaments (804), the bundle terminating at the light emitting end in a planar surface (808) oblique to the longitudinal axis (816), each filament (804) having an oblique end face (805) coplanar with the planar surface (808).

20. Apparatus according to any of claims 6 to 16,
**characterized in that**
the conduit comprises a single filament terminating at the light emitting end in a planar surface oblique to the longitudinal axis.

21. Apparatus according to claim 1,
**characterized in that**
the surgical retractor (210) has a retractor surface; and
the light conduit (500) is releasably coupled to the surgical retractor (210), the conduit (500) comprising a sheath (506) having an outer wall (508) terminating at an angled end portion, an opening (510) extending through the sheath wall (508) at the angled end, a plurality of light transmitting filaments (504) disposed within the sheath (506), the filaments (504) terminating in an end portion having a longitudinal axis (516) and an end face (514) normal to the axis (516) and contained within the sheath (506), the end portion being fully recessed within the retractor surface, the conduit (500) emitting light through the opening (510) along the longitudinal axis (516).

22. Apparatus according to claim 1,
**characterized in that**
the surgical retractor (210) has a retractor surface including an opening; and
the light conduit (800) is coupled to the retractor (210), the light conduit (800) comprising at least one light transmitting filament (804) terminating at the light emitting end having a longitudinal axis (816), the at least one filament (804) defining a planar surface (808) at the light emitting end oblique to the longitudinal axis (816), the planar surface (808) being positioned within the opening.

23. Apparatus according to claim 22,
**characterized in that**
the planar surface (808) is flush with the retractor surface (340).

24. Apparatus according to claim 22 or 23,
**characterized in that**
the light conduit (800) comprises a plurality of light transmitting filaments (804), each filament (804) terminating in an oblique end face (805) coplanar with the planar surface (808).
